# FASCICULE DE BREVET EUROPEEN

(11) **EP 0 301 034 B1**
(45) Date de publication et mention de la délivrance du brevet: **11.01.1995**
(21) Numéro de dépôt: 87907513.3
(22) Date de dépôt: 03.11.1987
(51) Int. Cl.: A61K 31/415

(54) **UTILISATION DE LA 1-(3'TRIFLUOROMETHYL 4'-NITROPHENYL) 4,4-DIMETHYL IMIDAZOLINE 2,5-DIONE POUR LA FABRICATION D'UN MEDICAMENT DESTINE AU TRAITEMENT DES CANCERS HORMONODEPENDANTS AUTRES QUE CEUX DE LA PROSTATE, DE LA PEAU ET DU COLON**
VERWENDUNG VON 1-(3'-TRIFLUORMETHYL-4'-NITROPHENYL)-4,4- DIMETHYLIMIDAZOLIN-2,5-DION ZUR HERSTELLUNG EINES MEDIKAMENTS ZUR BEHANDLUNG VON HORMONABHÄNGIGEN KREBSERKRANKUNGEN AUSSER DENEN DER PROSTATA, DER HAUT UND DES GRIMMDARMS
USE OF 1-(3'TRIFLUOROMETHYL 4'-NITROPHENYL) 4,4-DIMETHYL IMIDAZOLINE 2, 5-DIONE IN THE MANUFACTURE OF A MEDICAMENT FOR THE TREATMENT OF HORMONE-DEPENDENT CANCERS OTHER THAN CANCERS OF THE PROSTATE, THE SKIN AND THE COLON

(30) Priorité: 04.11.1986 IT 4861886
(43) Date de publication de la demande: 01.02.1989
(73) Titulaire: ROUSSEL-UCLAF, 75007 Paris (FR)
(72) Inventeur: MOGUILEWSKY, Martine, F-94130 Nogent-sur-Marne (FR); BOUTON, Marie-Madeleine, F-75012 Paris (FR)
(74) Mandataire: Bourgouin, André
(86) Numéro de dépôt international: FR8700435
(87) Numéro de publication internationale: WO8803404

(56) Documents cités:
- DE-A- 2 649 925
- Gynäkologe (1979), vol. 12(4), p.228-42
- J. Steroid Biochem. (1984) vol. 20, no.6B, p.1397, Abrégé no. A65
- Virchows Arch. (1982), vol.38, p.351-55
- Carcinogenesis (1984), vol. 5, p.609-14 Ann. Urol. (1986), vol. 20(2), p.98-106

## Description

La 1-(3'trifluorométhyl 4'-nitrophényl) 4,4-diméthyl imidazoline 2,5-dione de formule : 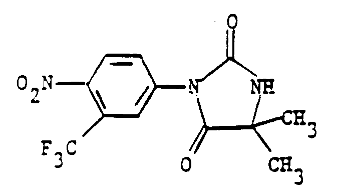
a été décrite dans le brevet français FR A 2 329 276 publié le 27 mai 1977.

L'application de ce produit au traitement des adénones et des noéplasies de la prostate a été décrite dans le même brevet.

On a ensuite décrit dans le brevet FR A 2 465 486 et dans de nombreuses publications telles que :
- (GYNAEKOLOGE, vol 12, n° 4, pp 228-242 (1979)), l'application de l'association entre la 1-(3'-trifluorométhyl 4'-nitrophényl) 4,4-diméthyl imidazoline et des peptides du type LH RH dans le traitement de l'adénocarcinome de la prostate et de l'hypertrophie bénigne de la prostate, de l'endométriose, de la dysménorrhée, de l'hirsutisme et des tumeurs mammaires hormonodépendantes.
- Virchows Arch. (1982), vol. 38, p.351-55 concerne une étude pharmacologique mettant en évidence l'activité inhibitrice d'un antiandrogène, le flutamide, sur la croissance des tumeurs du côlon.
- Carcinogenesis (1984), vol. 5, p. 609-14 concerne les effets inhibiteurs de l'acétate de cyprotérone sur la croissance des papillomes de la peau.

Par ailleurs, la présence de récepteurs androgènes a été décrite dans les cellules cancéreuses d'un certain nombre d'organes.

On peut citer par exemple les publications suivantes :
- tumeur de la vessie : Urology 1985, Fév. Vol. 25(2), pp. 161-3,
- tumeur du cerveau : J. Neurooncology 1983, Vol. 1(3), pp. 179-89,
- tumeur du sein : Cancer 1984, Déc. 1, Vol. 54(11), pp. 2436-2440,
- lymphome : J. Steroid Biochemistry, 1984, Oct., Vol. 21(4), pp. 421-6,
- tumeur du rein : Cancer 1984, August 1, Vol. 54(3), pp. 477-81,
- tumeur du foie : Br J. Cancer 1983, Déc., Vol. 48(6), pp. 791-6,
- mélanone : Br J. Dermatol. 1982, Nov., Vol. 107, suppl. 23, pp. 54-9,
- tumeur des ovaires : J. Endocrinol 1981, Sept. Vol. 90(3), pp. 421-31.

La publication précitée "Cancer 1984, August 1, Vol. 54(3), pp. 477-81" décrit l'absence d'activité du Flutamide (2-méthyl N-[4-nitro 3-(trifluorométhyl) phényl] propanamide) dans le carcinome rénal.

A la connaissance de la société demanderesse, l'application de la 1-(3'-trifluorométhyl 4'-nitrophényl) 4,4-diméthyl imidazoline 2,5-dione au traitement de cancers hormonodépendants autres que celui de la prostate, de la peau et du côlon n'a jamais été décrite.

La présente demande de brevet a donc pour objet l'utilisation de la 1-(3'trifluorométhyl 4'-nitrophényl) 4,4-diméthyl imidazoline 2,5-dione pour l'obtention d'un médicament destiné au traitement de cancers hormonodépendants autres que celui de la prostate, de la peau et du côlon.

Plus particulièrement, objet de la présente demande de brevet, concerne l'utilisation de la 1-(3'trifluorométhyl 4'-nitrophényl) 4,4-diméthyl imidazoline 2,5-dione pour l'obtention d'un médicament destiné au traitement du cancer affectant les organes suivants : vessie, cerveau, sein, système lymphatique, rein, foie et ovaires.

Plus particulièrement encore, l'objet de la présente demande de brevet, concerne l'utilisation de la 1-(3'-trifluorométhyl 4'-nitrophényl) 4,4-diméthyl imidazoline 2,5-dione pour l'obtention d'un médicament destiné au traitement du cancer du sein, du cerveau ou des ovaires.

Enfin, la présente demande concerne plus particulièrement l'utilisation de la 1-(3'-trifluorométhyl 4'-nitrophényl) 4,4-diméthyl imidazoline 2,5-dione pour l'obtention d'un médicament destiné au traitement du cancer du sein ou des ovaires.

Dans l'utilisation décrite ci-dessus, les médicaments comprenant , comme principe actif, la 1-(3'-trifluorométhyl 4'-nitrophényl) 4,4-diméthyl imidazoline 2,5-dione sont administrées par voie parentérale, buccale, perlinguale ou rectale. La voie préférée est la voie buccale ou percutanée.

Les médicaments utilisés peuvent se présenter sous forme de suspensions injectables, de comprimés, de comprimés enrobés, de capsules, de suppositoires, de préparations dermiques. Ces médicaments sont préparés de façon classique.

Les doses utilisables sont variables selon l'affection à traiter.

Elles peuvent par exemple varier de 0,5 à 20 mg/kg par jour par voie orale. De préférence, la dose employée varie de 1 à 5 mg/kg par jour.

L'activité de la 1-(3'-trifluorométhyl 4'-nitrophényl) 4,4-diméthyl imidazoline 2,5-dione a été déterminée par la mesure de son activité antiproliférative sur la croissance de cellules tumorales mammaires MCF-7 et T 47-D.

### Description du test :

### a) Culture cellulaire.

Les lignées MCF-7 et T 47-D sont maintenues en culture en milieu SVF (1) à 37^{¤}C en atmosphère humide contenant 5% CO₂. Les cellules à subconfluence sont récoltées par trypsination (trypsine 0,05%, EDTA 0,02%) puis rincées par centrifugation douce. Un échantillon des cellules en suspension est compté sur cellule de Malassez.

### b) Etude de la croissance.

Les cellules resuspendues dans le milieu SVF sont ensemencées à raison de 30.000 cellules par puits dans des plaques multipuits (24 puits de 2,5 cm2). Vingt quatre heures après l'ensemencement (JO), le produit à tester est ajouté au milieu en solution éthanolique (concentration finale en éthanol : 0,1%), à la concentration de 10⁻⁵M, les puits contrôles recevant la même concentration en éthanol. Les milieux sont renouvelés toutes les 48 heures. En fin d'expérience (J6 pour les cellules MCF-7 et J12 pour les T 47-D), le milieu est aspiré et les cellules sont immédiatement fixées par 150 microlitres de méthanol afin de doser l'ADN.

L'activité anti-proliférative des produits est évaluée par leur capacité à inhiber l'augmentation d'ADN.

### c) Dosage de l'ADN.

L'ADN est dosé par une méthode fluorimétrique utilisant le DABA (Acide 3,5 diaminobenzoïque) (2) 150 microlitres de DABA sont ajoutés dans chaque puits ; les plaques sont alors incubées 45 mn à 56°C, puis 1,5 ml d'HCl 1N sont ajoutés. La fluorescence est mesurée à l'aide de fluorimètre (longueur excitatrice : 400 nm, longueur d'onde d'émission : 500 nm). La quantité d'ADN par puits est évaluée par rapport à une gamme étalon obtenue en traitant dans les mêmes conditions un standard d'ADN du thymus de veau.

### Résultats :

A la concentration de 10⁻⁵M, la 1-(3'-trifluorométhyl 4'-nitrophényl) 4,4-diméthyl imidazoline 2,5-dione inhibe de 30%, la croissance des cellules MCF-7 et T 47-D.
(1) Le milieu de culture sérum de veau foetal (SVF) est préparé comme suit :
   Milieu MEM (minimal Essential Medium) auquel sont ajoutés :
   - acides aminés non essentiels (GIBCO),
   - péni-strepto (pénicilline 100U/ml, streptomycine 0,1 mg/ml),
   - fungizone 0,1%,
   - insuline (5 ng/ml),
   - sérum de veau foetal (4% concentration finale),
(2) Puzas et Goodman, Analytical Biochemistry, Vol. 86, pp. 50-50, 1978.

## Revendications

1. Utilisation de la 1-(3'trifluorométhyl 4'-nitrophényl) 4,4-diméthyl imidazoline 2,5-dione pour l'obtention d'un médicament destiné au traitement de cancers hormonodépendants autres que celui de la prostate, de la peau et du côlon.

2. Utilisation de la 1-(3'trifluorométhyl 4'-nitrophényl) 4,4-diméthyl imidazoline 2,5-dione pour l'obtention d'un médicament destiné au traitement du cancer affectant les organes suivants : vessie, cerveau, sein, système lymphatique, rein, foie et ovaires.

3. Utilisation de la 1-(3'-trifluorométhyl 4'-nitrophényl) 4,4-diméthyl imidazoline 2,5-dione pour l'obtention d'un médicament destiné au traitement du cancer du sein, du cerveau ou des ovaires.

4. Utilisation de la 1-(3'-trifluorométhyl 4'-nitrophényl) 4,4-diméthyl imidazoline 2,5-dione pour l'obtention d'un médicament destiné au traitement du cancer du sein ou des ovaires.

## Claims

1. Use of 1-(3'-trifluoromethyl 4'-nitrophenyl) 4,4-dimethyl imidazoline 2,5-dione for obtaining a medicament intended for the treatment of hormone-dependent cancers other than that of the prostate, the skin and the colon.

2. Use of 1-(3'-trifluoromethyl 4'-nitrophenyl) 4,4-dimethyl imidazoline 2,5-dione for obtaining a medicament intended for the treatment of cancer affecting the following organs: bladder, brain, breast, lymphatic system, kidney, liver and ovaries.

3. Use of 1-(3'-trifluoromethyl 4'-nitrophenyl) 4,4-dimethyl imidazoline 2,5-dione for obtaining a medicament intended for the treatment of cancer of the breast, brain or ovaries.

4. Use of 1-(3'-trifluoromethyl 4'-nitrophenyl) 4,4-dimethyl imidazoline 2,5-dione for obtaining a medicament intended for the treatment of cancer of the breast or of the ovaries.

## Patentansprüche

1. Verwendung von 1-(3'-Trifluormethyl-4'-nitrophenyl)-4,4-dimethylimidazolin-2,5-dion zur Herstellung eines Arzneimittels zur Behandlung hormonabhängiger Krebserkrankungen außer denen der Prostata, der Haut und des Grimmdarms.

2. Verwendung von 1-(3'-Trifluormethyl-4'-nitrophenyl)-4,4-dimethylimidazolin-2,5-dion zur Herstellung eines Arzneimittels zur Behandlung von Krebserkrankungen folgender Organe: Blase, Gehirn, Brust, lymphatisches System, Niere, Leber und Eierstöcke.

3. Verwendung von 1-(3'-Trifluormethyl-4'-nitrophenyl)-4,4-dimethylimidazolin-2,5-dion zur Herstellung eines Arzneimittels zur Behandlung von Krebserkrankungen der Brust, des Gehirns oder der Eierstöcke.

4. Verwendung von 1-(3'-Trifluormethyl-4'-nitrophenyl)-4,4-dimethylimidazolin-2,5-dion zur Herstellung eines Arzneimittels zur Behandlung von Krebserkrankungen der Brust oder der Eierstöcke.
